(19) Europäisches Patentamt
European Patent Office
Office européen des brevets

(11) Publication number: 0 080 220
B1

(12) EUROPEAN PATENT SPECIFICATION

(45) Date of publication of patent specification: 19.02.86

(21) Application number: 82201367.8

(22) Date of filing: 01.11.82

(51) Int. Cl.$^4$: C 07 D 413/04, C 07 D 417/04, C 07 D 491/04, A 61 K 31/445 // C07D271/08 ,(C07D491/04, 307:00, 221:00)

# (54) Dihydropyridines, methods for their production and their formulation and use as pharmaceuticals.

(30) Priority: 17.11.81 GB 8134550
01.09.82 GB 8224923

(43) Date of publication of application:
01.06.83 Bulletin 83/22

(45) Publication of the grant of the patent:
19.02.86 Bulletin 86/08

(84) Designated Contracting States:
AT BE CH DE FR GB IT LI LU NL SE

(56) References cited:
EP-A-0 000 150
EP-A-0 042 089
EP-A-0 071 819
EP-A-0 088 276
DE-A-2 629 892
FR-A-2 438 654
GB-A-1 591 089
GB-A-2 037 766
GB-A-2 041 358

The file contains technical information submitted after the application was filed and not included in this specification

(73) Proprietor: FISONS plc
Fison House Princes Street
Ipswich Suffolk IP1 1QH (GB)

(72) Inventor: Dixon, John
35 de Verdun Avenue
Belton Leicestershire (GB)
Inventor: Tinker, Alan Charles
97 Knighttthorpe Road
Loughborough Leicestershire (GB)

(74) Representative: Craig, Christopher Bradberry et al
Fisons plc 12 Derby Road
Loughborough Leicestershire LE11 0BB (GB)

EP 0 080 220 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new compounds, methods for their preparation and compositions containing them.

Dihydropyridine derivatives having in position 4 a benzofurazanyl or a benzothiadiazolyl substituent, in position 2 hydrogen or alkyl are for instance described in EP—A—150.

According to the invention we provide compounds of formula I,

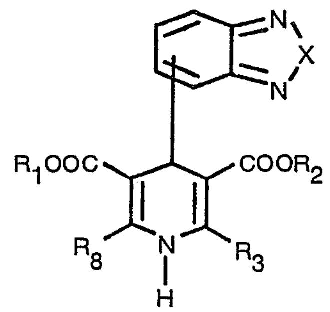

I

in which X is oxygen or sulphur,

$R_1$ and $R_2$, which may be the same or different, each represent alkyl C 1 to 6, $-(CH_2)_nOR_5$, or $-(CH_2)_nNR_6R_7$, and in addition $R_2$ may represent hydrogen,

n is a whole number from 2 to 4 inclusive,

$R_3$ represents $-CH_2OH$, —CN, $-CH(OR_4)_2$, —CHO, —CH=NOH, $-CF_3$, or, together with $R_2$ forms a chain —CHOH—,

$R_4$ represents alkyl C 1 to 6,

$R_5$ represents alkyl C1 to 6 or phenyl,

$R_6$ and $R_7$, which may be the same or different, each represent alkyl C1 to 6 optionally substituted by phenyl, and

$R_8$ is alkyl C1 to 6,

and pharmaceutically acceptable acid addition salts of those compounds containing a basic nitrogen atom.

According to the invention we also provided a process for the production of a compound of formula I, or a pharmaceutically acceptable acid addition salt thereof, which comprises

a) production of a compound of formula I in which $R_3$ is $-CH(OR_4)_2$ by

i) reaction of a compound of formula II,

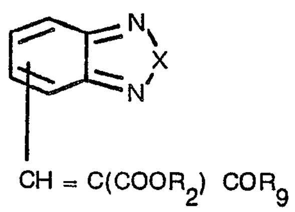

II

in which $R_9$ is a group $-CF_3$ or $-CH(OR_4)_2$, X, $R_2$ and $R_4$ are as defined above, with a compound of formula III,

$$R_1OOCCH=C(R_8)NH_2 \qquad III$$

in which $R_1$ and $R_8$ are as defined above,

or ii) reaction of a corresponding compound of formula I in which $R_3$ is —CHO with an alcohol $R_4OH$,

b) production of a compound of formula I in which $R_3$ is —CHO by selective hydrolysis of a corresponding compound of formula I in which $R_3$ is $-CH(OR_4)_2$,

c) production of a compound of formula I in which $R_3$ is —CH=NOH by reaction of a corresponding compound of formula I in which $R_3$ is —CHO with hydroxylamine, or a salt thereof,

d) production of a compound of formula I in which $R_3$ is —CN by dehydration of a corresponding compound of formula I in which $R_3$ is —CH=NOH,

e) production of a compound of formula I in which $R_3$ is $-CH_2OH$ by selective reduction of a corresponding compound of formula I in which $R_3$ is —CHO,

f) production of a compound of formula I in which $R_2$ is hydrogen, by selective hydrolysis of a corresponding compound of formula I in which $-COOR_2$ is an ester group,

g) production of a compound of formula I in which $R_2$ is other than hydrogen by esterification, with a

compound $R_2L$ in which L is a leaving group, of a corresponding compound of formula I in which $R_2$ is hydrogen, or $R_2$ together with $R_3$ forms a chain —CHOH—, or

h) selective reduction of a corresponding pyridine,

and where desired or necessary converting the resulting compound of formula I to a pharmaceutically acceptable acid addition salt thereof, or vice versa.

Process a) i) may be carried out at a temperature of from about 50 to 150°C. The reaction may be carried out in an excess of the compound of formula III, or, less preferably, in a solvent which is inert under the reaction conditions, e.g. a polar solvent such as methanol, ethanol, dioxan or dimethylformamide. We prefer $R_2$ to be methyl or ethyl.

Process a) ii) may be carried out in the presence of an excess of the alcohol and preferably in the presence of an acidic catalyst. The reaction may be carried out at a temperature of from about 50 to 150°C.

In processes a) i) and a) ii) we prefer $R_2$ not to be hydrogen.

The hydrolysis of process b) may be carried out using an aqueous acid, for example hydrochloric acid (e.g. 0.5 to 2.5 molar), in a water miscible organic solvent, e.g. acetone or tetrahydrofuran. The reaction may be carried out at a temperature of from about 20 to 50°C. The reaction is preferably not carried out for more than about 6 hours.

Process c) may be carried out in a suitable solvent, e.g. acetic acid or ethanol, and in the presence of a basic catalyst, e.g. sodium acetate or sodium carbonate. The reaction may be carried out at a temperature of from about 20 to 120°C.

The dehydration of process d) may be carried out using a variety of dehydrating agents, e.g. an excess of acetic anhydride, thionyl chloride in ether or N,N'-dicyclohexylcarbodiimide in pyridine. The reaction may be carried out at a temperature of from about 0 to 150°C depending on the reagent and solvent used.

The reduction of process e) may be carried out either chemically or catalytically, e.g. by use of sodium borohydride in an alcoholic solvent, e.g. methanol or ethanol at a temperature of from about 0 to 50°C.

Process f) may be carried out under similar, but comparatively more forcing, conditions to process b) above, e.g. temperature of up to 100°C, reaction time of 24 to 72 hours and stronger acid, e.g. 2.5 to 5 molar HCl. We prefer the process to be carried out using a starting material in which $R_3$ is —CHO. Processes b) and f) may be carried out simultaneously under the more forcing conditions of process f).

In process g) the leaving group is preferably an anion forming group, e.g. chlorine, bromine, iodine, methanesulphonate or *p*-toluenesulphonate. The reaction may be carried out at a temperature of from about 20 to 100°C and in a polar solvent which is inert under the reaction conditions, e.g. acetone, acetonitrile or dimethylformamide and in the presence of a base, e.g. sodium or potassium carbonate.

The reduction of process h) may be carried out using an appropriate reducing agent, e.g. a dithionate such as sodium dithionate, in an aqueous organic solvent, for example aqueous ethanol or aqueous dioxan. The reaction may be carried out at a temperature of from about 20° to 80°C.

In the above processes where $R_2$ is $-(CH_2)_nNR_6R_7$ the reaction may be carried out using, and may, where appropriate produce, a compound in the form of an acid addition salt thereof. Such processes are included within the scope of the invention.

Compounds of formula II may be made by the reaction of a compound of formula IV,

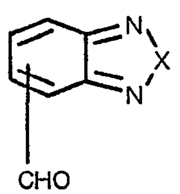

IV

in which X is as defined above, with a compound of formula V,

$$R_2OOCCH_2COR_9 \qquad V$$

in which $R_2$ and $R_9$ are as defined above.

The reaction may be carried out in refluxing benzene or toluene in the presence of an organic base or an anhydrous acid, the water produced being removed by azeotropic distillation.

Alternatively compounds of formula II may be made by reaction of a compound of formula V with a compound of formula VI,

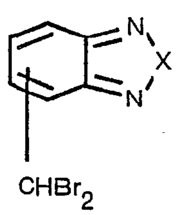

VI

in which X is as defined above,
in the presence of a basic catalyst.

Compounds of formulae III, IV, V, and VI and the pyridine starting materials for process h) are either known or may be made from known compounds using conventional techniques known *per se*. The pyridine starting materials for process h) may also be made by selective oxidation, e.g. using dichlorodicyano-benzoquinone in an organic solvent such as chloroform, of the corresponding dihydropyridine of formula I.

The compounds of formula I, and the intermediates therefor, may be isolated from their reaction mixtures using conventional processes known *per se*.

The compounds of formula I possess an asymetric carbon atom and the invention provides the compounds in optically active form or as racemic or other mixtures. The optical isomers may be separated by conventional procedures which are known *per se*.

We prefer X to be oxygen. We also prefer $R_1$, $R_2$ and $R_4$ to be selected from methyl, and, more preferably, ethyl and isopropyl. In particular $R_1$ and $R_2$ can both be ethyl or methyl, or one can be methyl or ethyl and the other isopropyl. n is preferably 2. $R_3$ is preferably $-CH_2OH$ or especially $-CHO$ or $-CN$. We also prefer the benzofurazanyl or benzothiadiazolyl group to be attached to the tetrahydropyridine moiety through its 4-position. $R_8$ is preferably C1 to 4 and most preferably is methyl. Compounds of formula I containing a basic nitrogen atom include those in which $R_2$ is $-(CH_2)NR_6R_7$. We prefer the compounds of Examples 3, 4 ii), 7, 10 ii), 10 iii), 12 ii), 12 iii) and particularly 11.

The compounds of formula I, and the pharmaceutically acceptable salts thereof, are useful because they possess pharmacological properties in animals. More particularly they block the entry of calcium into vascular and cardiac muscle leading to a fall in blood pressure, inotropy and heart rate. They are active in the following systems:—

a) Relaxation of contracted vascular smooth muscle. Van Breemen, Aaronson, Loutzenhiser and Meisheri, Chest, *78*, Supplement, 157—165, 1980.

(b) Reduce inotropy and chronotropy of isolated atria. Henry, Excerpta Med. Int. Congr. Ser., *474*, 14—23, 1979.

(c) Reduce blood pressure and increase cardiac output in anaesthetised dogs. Hirakawa, Ito, Kondo, Watanbe, Hiei, Banno & Hyase, Arzneim-Forsch, *22*, 344—349, 1972.

(d) Reduce blood pressure with small rises in heart rate in conscious dogs when given by the intravenous and oral routes. Newman, Bishop, Peterson, Leroux & Horowitz, J. Pharm. Exp. Ther. *201*, 723—730, 1977.

The compounds are thus indicated for use in the treatment of hypertension, angina pectoris, variant angina, congestive heart failure, myocardial infarction, cerebrovascular disorders, vascular disease and in asthmatic conditions.

For the above uses the dosage will depend upon the compound used, the route of administration and the effect desired, but in general will be in the range of 0.1—10 mg per kilogram body weight per day. For man the indicated total daily dose will be from about 5—500 mg, preferably from 5 to 200 mg and more preferably from 5 to 100 mg, which may be administered in divided doses of about 1—200 mg, preferably 2 to 25 mg, e.g. 2 to 4 times per day.

The compounds of formula I are advantageous in that they possess greater potency (e.g. with respect to direct negative chronotropic effect), produce a lower level of reflex tachycardia, are more selective (e.g. for vascular smooth muscle vs cardiac muscle), produce less depression of cardiac contractility, are longer acting, are more easily formulated, possess less, or less undesirable, side effects or have other more beneficial properties than known compounds of similar structure.

The compounds of the invention may be administered by a wide variety of routes and may act systemically or locally. Thus the compounds may be administered by oral or nasal inhalation to the lung, to the buccal cavity, oesophageally, rectally, topically to the skin or to other available surfaces of the body; by injection, e.g. intravenously, intramuscularly, intraperitoneally, or surgical implant.

According to our invention we also provide a pharmaceutical composition comprising preferably less than 80% and more preferably less than 50% by weight of a compound of formula I, or a pharmaceutically acceptable salt thereof, in admixture with a pharmaceutically acceptable adjuvant, diluent or carrier. Examples of suitable adjuvants, diluents or carriers are: for tablets, capsules and dragées; microcrystalline cellulose, polyethylene glycol, calcium phosphate, diatomaceous earth, a sugar such as lactose, dextrose or mannitol, talc, stearic acid, starch, sodium bicarbonate and/or gelatin;

for suppositories; natural or hardened oil or waxes; and

for inhalation compositions, coarse lactose.

The compounds may also be formulated in controlled release form, e.g. in the form of a matrix out of which the compound is slowly leached.

The compounds may also, if desired, be used in admixture with other pharmaceutically active compounds, e.g. a beta blocker.

Compounds of formula I in which $R_2$ is hydrogen and $R_3$ is $-CHO$ may exist in the tautomeric form Ia,

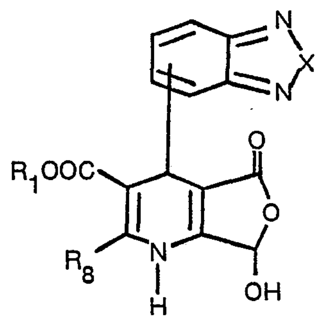

Ia

i.e. a form in which $R_3$ together with $R_2$ forms a chain —CHOH—.

The invention is illustrated, but in no way limited by the following Examples in which temperatures are in degrees centigrade.

Example 1

Diethyl 4-(4-benzofurazanyl)-2-(diethoxymethyl)-1,4-dihydro-6-methylpyridine-3,5-dicarboxylate

A solution of 4-benzofurazancarboxaldehyde (6.0 g, 40 mmoles), ethyl 4,4-diethoxy-3-oxobutyrate (8.0 g, 37 mmoles) and piperidine (0.5 ml) in dry benzene (150 ml) was heated at reflux using a Dean and Stark water separator for 3 hours. The cooled solution was added to ether (200 ml) and the organic solution washed with water and brine, and then dried ($MgSO_4$). Evaporation of the solvents left crude ethyl 2-(4-benzofurazanmethylidene)-4,4-diethoxy-3-oxobutanoate (mixture of E- and Z-isomers) as a brown oil (13.5 g).

The foregoing oil (13.5 g, 37 mmoles) and ethyl 3-amino-2-butenoate (4.76 g, 37 mmoles) were mixed and heated at 100°C for 16 hours. The resulting dark oil was dissolved in ether (200 ml) and the organic solution washed with water and brine, and then dried ($MgSO_4$). Evaporation of the solvent left the title compound as an oil (18.8 g) $M^+$ 459.

Example 2

By the method of Example 1 were also prepared

i) Dimethyl 4-(4-benzofurazanyl)-1,4-dihydro-2-(dimethoxymethyl)-6-methylpyridine-3,5-dicarboxylate (Oil, $M^+$ 403)

ii) 3-Ethyl 5-(1-methylethyl)-4-(4-benzofurazanyl)-2-(diethoxymethyl-1,4-dihydro-6-methylpyridine-3,5-dicarboxylate (Oil, $M^+$ 473)

iii) Diethyl 4-[4-(2,1,3-benzothiadiazolyl)]-2-(diethoxymethyl)-1,4-dihydro-6-methylpyridine-3,5-dicarboxylate (Oil, $M^+$ 475)

Example 3

Diethyl 4-(4-benzofurazanyl)1,4-dihydro-2-formyl-6-methylpyridine-3,5-dicarboxylate

The product of Example 1 above (18.8 g) in tetrahydrofuran (100 ml) was treated with 50% hydrochloric acid (25 ml). After 4 hours at room temperature the solvent was removed by evaporation to leave a brown gum which was dissolved in ethyl acetate (150 ml) and treated with saturated aqueous sodium bicarbonate until pH 9.

The organic solution was separated and washed with sodium bicarbonate solution, water and brine, and then dried ($MgSO_4$). Evaporation of the solvent left a brown oil (16.1 g), which gave a solid on trituration with 2-propanol. Recrystallisation from 2-propanol gave the title compound (6.1 g) as an orange solid m.p. 131—132°.

Example 4

By the method of Example 3 were also prepared

i) Dimethyl 4-(4-benzofurazanyl)-1,4-dihydro-2-formyl-6-methylpyridine-3,5-dicarboxylate m.p. 166.5—167.5°.

ii) 3-Ethyl 5-(1-methylethyl)4-(4-benzofurazanyl)-1,4-dihydro-2-formyl-6-methylpyridine-3,5-dicarboxylate m.p. 115—116°.

iii) Diethyl 4-[4-(2,1,3-benzothiadiazolyl)]-1,4-dihydro-2-formyl-6-methylpyridine-3,5-dicarboxylate m.p. 122.5—124°.

iv) 3-Ethyl 5-(2-methylpropyl)4-(4-benzofurazanyl)-1,4-dihydro-2-formyl-6-methylpyridine-3,5-dicarboxylate m.p. 97—98°.

v) 3-Ethyl 5-(2-(N-methyl-N-(phenylmethyl)amino)ethyl)4-(4-benzofurazanyl)-1,4-dihydro-2-formyl-6-methylpyridine-3,5-dicarboxylate hydrochloride m.p. 115°.

Example 5

3-Ethyl 5-(1-methylethyl) 4-(4-benzofurazanyl)-1,4-dihydro-6-methyl-2-[bis(1-methylethoxy)-methyl]pyridine-3,5-dicarboxylate

The product of Example 4 (ii) above (5.0 g, 12.5 mmoles) in 2-propanol (100 ml) was heated at reflux for 45 minutes. The solvent was removed by evaporation and the resulting yellow solid separated on silica (200 g) using ether-petroleum ether mixtures as eluent. The first eluted band, after removal of solvent and recrystallisation from 2-propanol, gave the title compound as a yellow solid (2.2 g) m.p. 103—104.5°.

Example 6

4-(4-Benzofurazanyl)-1,4-dihydro-2-formyl-6-methylpyridine-3,5-dicarboxylic acid, 5-methyl ester

A solution of the product of Example 4 (i) (5.0 g, 12.4 mmoles) in tetrahydrofuran (50 ml) was treated with 50% hydrochloric acid (25 ml). After 72 hours at room temperature the solvent was removed by evaporation to leave a gum which was dissolved in ethyl acetate, and neutralised with sodium bicarbonate. The organic solution was separated and washed with water and brine, and then dried ($MgSO_4$). Evaporation of the solvent gave a yellow solid (3.2 g) m.p. 224.5°—225.5° (decompn.).

NMR analysis indicates that this compound exists almost entirely in the cyclised pseudo-acid form, viz: Methyl 4-(4-benzofurazanyl)-1,4,5,7-tetrahydro-7-hydroxy 5-oxo-2-methylfuro[3,4-b]pyridine-3-carboxylate, when in solution.

Example 7

5-Methyl 3-(1-methylethyl) 4-(4-benzofurazanyl)-1,4-dihydro-2-formyl-6-methylpyridine-3,5-dicarboxylate

The product of Example 6 above (2.22 g, 6.4 mmoles), anhydrous potassium carbonate (1.78 g, 12.8 mmoles) and 2-iodopropane (0.8 ml, 8 mmoles) in acetonitrile (100 ml) were heated at reflux for 3 hours. The cooled mixture was filtered, the solid washed with ethyl acetate and the combined filtrates concentrated *in vacuo*. The resulting oil was dissolved in ethyl acetate (100 ml) and the solution washed with water and brine, and then dried ($MgSO_4$). Evaporation of the solvent left a brown oil which was separated by chromatography on silica (100 g) using ether-petroleum ether mixtures as eluent. The title compound (0.9 g) was obtained as an orange solid m.p. 166—167° after trituration with petroleum ether (b.p. 40—60°).

Example 8

By the method described in Example 7 above was also prepared:

i) 5-Methyl 3-(2-phenoxyethyl)4-(4-benzofurazanyl)-1,4-dihydro-2-formyl-6-methylpyridine-3,5-dicarboxylate m.p. 121—122°.

ii) 3-(2-Ethoxyethyl)5-methyl 4-(4-benzofurazanyl)-1,4-dihydro-2-formyl-6-methylpyridine-3,5-dicarboxylate m.p. 142°.

iii) 3-(2-(N,N-diethylamino)ethyl)5-methyl 4-(4-benzofurazanyl)-1,4-dihydro-2-formyl-6-methylpyridine-3,5-dicarboxylate hydrochloride m.p. 130°.

Example 9

Diethyl 4-(4-benzofurazanyl)-1,4-dihydro-2-(hydroxymethyl)-6-methylpyridine-3,5-dicarboxylate

A solution of the product of Example 3 above (3.6 g, 9.3 mmoles) in dry ethanol (100 ml) was cooled to 0° and sodium borohydride (0.42 g, 11 mmoles) was added portionwise over 3 minutes. After 30 minutes 10% aqueous hydrochloric acid was added dropwise to pH 3, and the mixture concentrated *in vacuo* at room temperature. The resulting yellow oil was dissolved in ethyl acetate (100 ml) and saturated aqueous sodium bicarbonate was added to pH 9. The organic solution was separated, washed with sodium bicarbonate solution, water and brine, and then dried ($MgSO_4$). Evaporation of the solvent left a yellow brown solid which was recrystallised from a mixture of ethyl acetate, ether and petroleum ether (b.p. 60—80°) to give the title compound as a yellow powder (1.47 g) m.p. 128—129°.

Example 10

By the method described in Example 9 above were also prepared

i) Dimethyl 4-(4-benzofurazanyl)-1,4-dihydro-2-(hydroxymethyl)-6-methylpyridine-3,5-dicarboxylate m.p. 182—184°.

ii) 3-Ethyl 5-(1-methylethyl) 4-(4-benzofurazanyl)-1,4-dihydro-2-(hydroxymethyl)-6-methylpyridine-3,5-dicarboxylate m.p. 118—119°.

iii) Diethyl 4-[4-(2,1,3-benzothiadiazolyl)]-1,4-dihydro-2-(hydroxymethyl)-6-methylpyridine-3,5-dicarboxylate m.p. 133.5—135°.

iv) 3-Ethyl 5-(2-methylpropyl) 4-(4-benzofurazanyl)-1,4-dihydro-2-(hydroxymethyl)-6-methylpyridine-3,5-dicarboxylate m.p. 99—101°.

Example 11

Diethyl 4-(4-benzofurazanyl)-2-cyano-1,4-dihydro-6-methylpyridine-3,5-dicarboxylate

A mixture of the product of Example 3 above (5.0 g, 13 mmoles), sodium acetate (2.13 g, 26 moles), hydroxylamine hydrochloride (1.13 g, 16.2 mmoles) and acetic acid was stirred at room temperature for 2 hours. Acetic anhydride (2.5 ml, 28.6 mmoles) was then added and the resulting mixture heated at reflux for 2 hours and then concentrated *in vacuo*. The yellow semi-solid residue was dissolved in ethyl acetate, and sodium bicarbonate added to pH 9. The organic solution was separated and washed with aqueous sodium bicarbonate, water and brine, and then dried ($MgSO_4$). Evaporation of solvent left a brown oil

which was purified by chromatography on silica (200 g) using ether-petroleum ether mixtures as eluent. The title compound was thus obtained as a yellow solid (1.12 g) m.p. 157—158° after recrystallisation from cyclohexane-ether.

Example 12

By the method of Example 11 above were also prepared:

i) Dimethyl 4-(4-benzofurazanyl)-2-cyano-1,4-dihydro-6-methylpyridine-3,5-dicarboxylate m.p. 231°.

ii) 3-Ethyl 5-(1-methylethyl) 4-(4-benzofurazanyl)-2-cyano-1,4-dihydro-6-methylpyridine-3,5-dicarboxylate m.p. 185—186.5°.

iii) Diethyl 4-[4-(2,1,3-benzothiadiazolyl)]-2-cyano-1,4-dihydro-6-methylpyridine-3,5-dicarboxylate m.p. 137—138°.

iv) 3-Ethyl 5-(2-methylpropyl) 4-(4-benzofurazanyl)-2-cyano-1,4-dihydro-6-methylpyridine-3,5-dicarboxylate m.p. 134—135°.

Example 13

3-Ethyl 5-(2-methylpropyl)4-(4-benzofurazanyl)-1,4-dihydro-2-(hydroxyiminomethyl)-6-methylpyridine-3,5-dicarboxylate

The product of Example 4 (iv) (5.4 g, 0.013 moles), hydroxylamine hydrochloride (1.18 g, 0.016 mole) and sodium acetate (2.15 g, 0.026 moles) in acetic acid were stirred at 20° for 2 hours. The solvent was evaporated, the residue taken up in ethyl acetate, and sodium bicarbonate added to pH 8. The organic phase was separated, washed with water, dried and evaporated. The residue was recrystallised from 2-propanol/petroleum ether and then from pure 2-propanol to give the title oxime as yellow crystals m.p. 167—8°.

Example 14

Diethyl 4-(4-benzofurazanyl)-1,4-dihydro-2-methyl-6-(trifluoromethyl)-pyridine-3,5-dicarboxylate

A solution of 4-benzofurazancarboxaldehyde (1.0 g, 6.75m moles), ethyl 4,4,4-trifluoro-3-oxybutyrate (1.0 ml., 6.75m moles) and piperidine (0.1 ml) in benzene (50 ml) was heated at reflux under a Dean-Stark water separator for 2 hours. The solution was cooled, ether (50 ml) added, and the mixture washed with water and brine and dried. Evaporation gave crude ethyl 2-(4-benzofurazanmethylidene)-4,4,4-trifluoromethyl-3-oxobutanoate (mixture of E- and Z-isomers) as a brown oil (2.2 g).

Ethyl 3-amino-2-butenoate (0.87 g, 6.75m moles) was added to the foregoing oil and the mixture heated on a steam bath for 72 hours. The mixture was cooled, dissolved in dichloromethane (50 ml) and trifluoroacetic anhydride (2.6 ml., 18.4m moles) and pyridine (1.6 ml., 18.4m moles) added. The solution was kept at 20° for 16 hours, then washed with water, aqueous hydrochloric acid, and brine. Evaporation of the organic phase afforded a brown oil (2.6 g), which was dissolved in ether and petroleum ether was added until precipitation of dark impurities was complete. The mixture was filtered and the filtrate cooled at 0° overnight to afford yellow crystals (0.5 g) of the title compound m.p. 100°.

**Claims for the Contracting States: BE CH DE FR GB IT LI LU NL SE**

1. A compound of formula I,

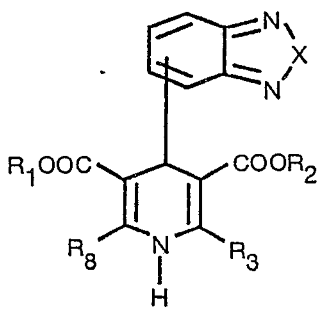

I

in which X is oxygen or sulphur,

$R_1$ and $R_2$, which may be the same or different, each represent alkyl C 1 to 6, $—(CH_2)_nOR_5$, or $—(CH_2)_nNR_6R_7$, and in addition $R_2$ may represent hydrogen,

n is a whole number from 2 to 4 inclusive,

$R_3$ represents $—CH_2OH$, —CN, $—CH(OR_4)_2$, —CHO, —CH=NOH, $—CF_3$, or, together with $R_2$ forms a chain —CHOH—,

$R_4$ represents alkyl C 1 to 6,

$R_5$ represents alkyl C1 to 6 or phenyl,

$R_6$ and $R_7$, which may the same or different, each represent alkyl C1 to 6 optionally substituted by phenyl, and

$R_8$ is alkyl C1 to 6,

and pharmaceutically acceptable acid addition salts of those compounds containing a basic nitrogen atom.

2. A compound according to Claim 1 in which X is oxygen and the benzofurazanyl group is attached through its 4-position to the tetrahydropyridine ring.

3. A compound according to Claim 1 or 2, wherein $R_1$ and $R_2$ are selected from ethyl and isopropyl.

4. A compound according to any one of the preceding claims, wherein $R_3$ is $—CH_2OH$, —CHO or —CN.

5. A compound according to Claim 4, wherein $R_3$ is —CHO or —CN.

6. A compound according to any one of the preceding claims, wherein $R_8$ is methyl.

7. A compound according to Claim 1, wherein the benzofurazanyl or benzothiadiazolyl group is attached to the dihydropyridine moiety through its 4-position,

$R_1$ and $R_2$, which may be the same or different, each represent alkyl C1 to 6, and

$R_3$ represents $—CH_2OH$, —CN, $—CH(OR_4)_2$, —CHO or —CH=NOH, and

$R_8$ is methyl.

8. A compound according to Claim 1, wherein the benzofurazanyl or benzothiadiazolyl group is attached to the dihydropyridine moiety through its 4-position,

$R_3$ represent $—CH_2OH$, —CN, $—CH(OR_4)_2$, —CHO, —CH=NOH, or, together with $R_2$ forms a chain —CHOH—, and

$R_8$ is methyl,

and pharmaceutically acceptable salts of those compounds containing a group $—(CH_2)_nNR_6R_7$.

9. A compound according to Claim 7, wherein X is oxygen, and $R_3$ is —CN, —CH=NOH, —CHO or $—CH(OR_4)_2$.

10. A compound according to Claim 7, wherein X is oxygen, and $R_3$ is —CN.

11. A compound according to Claim 7, wherein X is oxygen, $R_3$ is —CN, one of $R_1$ and $R_2$ is methyl and the other is isopropyl.

12. Diethyl 4-(4-benzofurazanyl)-2-(diethoxymethyl)-1,4-dihydro-6-methylpyridine-3,5-dicarboxylate,

Dimethyl 4-(4-benzofurazanyl)-1,4-dihydro-2-(dimethoxymethyl)-5-methylpyridine-3,5-dicarboxylate,

3-Ethyl 5-(1-methylethyl)-4-(4-benzofurazanyl)-2-(diethoxymethyl)-1,4-dihydro-6-methylpyridine-3,5-dicarboxylate,

Diethyl 4-[4-(2,1,3-benzothiadiazolyl)]-2-(diethoxymethyl)-1,4-dihydro-6-methylpyridine-3,5-dicarboxylate,

Diethyl 4-(4-benzofurazanyl)-1,4-dihydro-2-formyl-6-methylpyridine-3,5-dicarboxylate,

Dimethyl 4-(4-benzofurazanyl)-1,4-dihydro-2-formyl-6-methylpyridine-3,5-dicarboxylate,

3-Ethyl 5-(1-methylethyl) 4-(4-benzofurazanyl)-1,4-dihydro-2-formyl-6-methylpyridine-3,5-dicarboxylate,

Diethyl 4-[4-(2,1,3-benzothiadiazolyl)]-1,4-dihydro-2-formyl-6-methylpyridine-3,5-dicarboxylate,

3-Ethyl 5-(2-methylpropyl) 4-(4-benzofurazany)1,4-dihydro-2-formyl-6-methylpyridine-3,5-dicarboxylate,

3-Ethyl 5-(2-(N-methyl-N-(phenylmethyl)amino)ethyl)4-(4-benzofurazanyl)-1,4-dihydro-2-formyl-6-methylpyridine-3,5-dicarboxylate hydrochloride,

3-Ethyl 5-(1-methylethyl) 4-(4-benzofurazanyl)-1,4-dihydro-6-methyl-2-[bis(1-methylethoxy)-methyl]pyridine-3,5-dicarboxylate,

4-(4-Benzofurazanyl)-1,4-dihydro-2-formyl-6-methylpyridine-3,5-dicarboxylic acid, 5-methyl ester,

Methyl 4-(4-benzofurazanyl)-1,4,5,7-tetrahydro-7-hydroxy 5-oxo-2-methylfuro[3,4-b]pyridine-3-carboxylate,

5-Methyl 3-(1-methylethyl) 4-(4-benzofurazanyl)-1,4-dihydro-2-formyl-6-methylpyridine-3,5-dicarboxylate,

5-Methyl 3-(2-phenoxyethyl) 4-(4-benzofurazanyl)-1,4-dihydro-2-formyl-6-methylpyridine-3,5-dicarboxylate,

3-(2-Ethoxyethyl) 5-methyl 4-(4-benzofurazanyl)-1,4-dihydro-2-formyl-6-methylpyridine-3,5-dicarboxylate,

3-(2-(N,N-diethylamino)ethyl) 5-methyl 4-(4-benzofurazanyl)-1,4-dihydro-2-formyl-6-methylpyridine-3,5-dicarboxylate hydrochloride,

Diethyl 4-(4-benzofurazanyl)-1,4-dihydro-2-(hydroxymethyl)-6-methylpyridine-3,5-dicarboxylate,

Dimethyl 4-(4-benzofurazanyl)-1,4-dihydro-2-(hydroxymethyl)-6-methylpyridine-3,5-dicarboxylate,

3-Ethyl 5-(1-methylethyl) 4-(4-benzofurazanyl)-1,4-dihydro-2-(hydroxymethyl)-6-methylpyridine-3,5-dicarboxylate,

Diethyl 4-[4-(2,1,3-benzothiadiazolyl)]-1,4-dihydro-2-(hydroxymethyl)-6-methylpyridine-3,5-dicarboxylate,

3-Ethyl 5-(2-methylpropyl) 4-(4-benzofurazanyl)-1,4-dihydro-2-(hydroxymethyl)-6-methylpyridine-3,5-dicarboxylate,

Diethyl 4-(4-benzofurazanyl)-2-cyano-1,4-dihydro-6-methylpyridine-3,5-dicarboxylate,

Dimethyl 4-(4-benzofurazanyl)-2-cyano-1,4-dihydro-6-methylpyridine-3,5-dicarboxylate,

3-Ethyl 5-(1-methylethyl) 4-(4-benzofurazanyl)-2-cyano-1,4-dihydro-6-methylpyridine-3,5-dicarboxylate,

Diethyl 4-[4-(2,1,3-benzothiadiazolyl)]-2-cyano-1,4-dihydro-6-methylpyridine-3,5-dicarboxylate,

3-Ethyl 5-(2-methylpropyl) 4-(4-benzofurazanyl)-2-cyano-1,4-dihydro-6-methylpyridine-3,5-dicarboxylate,

3-Ethyl 5-(2-methylpropyl) 4-(4-benzofurazanyl)-1,4-dihydro-2-(hydroxyiminomethyl)-6-methylpyridine-3,5-dicarboxylate, or

Diethyl 4-(4-benzofurazanyl)-1,4-dihydro-2-methyl-6-(trifluoromethyl)-pyridine-3,5-dicarboxylate.

13. A pharmaceutical formulation comprising a compound according to any one of the preceding claims in admixture with a pharmaceutically acceptable adjuvant, diluent or carrier.

14. A process for the production of a compound of formula I as defined in Claim 1, or a pharmaceutically acceptable acid addition salt thereof, which comprises

a) production of a compound of formula I in which $R_3$ is $—CH(OR_4)_2$ or $—CF_3$ by

i) reaction of a compound of formula II,

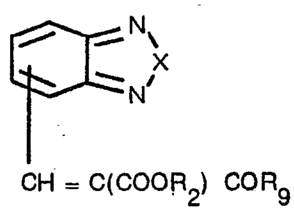

II

in which $R_9$ is a group $—CF_3$ or $—CH(OR_4)_2$, and X, $R_2$ and $R_4$ are as defined above, with a compound of formula III,

$$R_1OOCCH{=}C(R_8)NH_2 \qquad III$$

in which $R_1$ and $R_8$ are as defined above, or

ii) when $R_3$ in the product is to be $—CH(OR_4)_2$ reaction of a corresponding compound of formula I in which $R_3$ is —CHO with an alcohol $R_4OH$,

b) production of a compound of formula I in which $R_3$ is —CHO by selective hydrolysis of a corresponding compound of formula I in which $R_3$ is $—CH(OR_4)_2$,

c) production of a compound of formula I in which $R_3$ is —CH=NOH by reaction of a corresponding compound of formula I in which $R_3$ is —CHO with hydroxylamine, or a salt thereof,

d) production of a compound of formula I in which $R_3$ is —CN by dehydration of a corresponding compound of formula I in which $R_3$ is —CH=NOH,

e) production of a compound of formula I in which $R_3$ is $—CH_2OH$ by selective reduction of a corresponding compound of formula I in which $R_3$ is —CHO,

f) production of a compound of formula I in which $R_2$ is hydrogen, by selective hydrolysis of a corresponding compound of formula I in which $—COOR_2$ is an ester group,

g) production of a compound of formula I in which $R_2$ is other than hydrogen by esterification, with a compound $R_2L$ in which L is a leaving group, of a corresponding compound of formula I in which $R_2$ is hydrogen, or $R_2$ together with $R_3$ forms a chain —CHOH—, or

h) selective reduction of a corresponding pyridine,

and where desired or necessary converting the resulting compound of formula I to a pharmaceutically acceptable acid addition salt thereof, or vice versa.

15. A compound according to any one of Claims 1 to 12 for use as a pharmaceutical.

16. Use of a compound according to any one of Claims 1 to 12 for manufacture of a medicament for the treatment of hypertension, angina pectoris, variant angina, congestive heart failure, myocardial infarction, cerebrovascular disorder, vascular disease or asthma.

**Claims for the Contracting State: AT**

1. A process for the production of a compound of formula I,

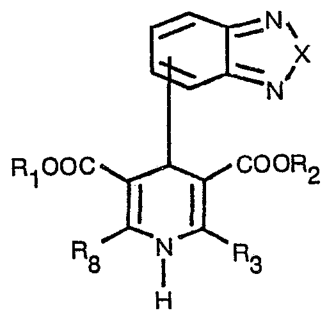

I

in which X is oxygen or sulphur,

$R_1$ and $R_2$, which may be the same or different, each represent alkyl C 1 to 6, $-(CH_2)_nOR_5$, or $-(CH_2)_nNR_6R_7$, and in addition $R_2$ may represent hydrogen,

n is a whole number from 2 to 4 inclusive,

$R_3$ represents $-CH_2OH$, $-CN$, $-CH(OR_4)_2$, $-CHO$, $-CH{=}NOH$, $-CF_3$, or, together with $R_2$ forms a chain $-CHOH-$,

$R_4$ represents alkyl C 1 to 6,

$R_5$ represents alkyl C1 to 6 or phenyl,

$R_6$ and $R_7$, which may the same or different, each represent alkyl C1 to 6 optionally substituted by phenyl, and

$R_8$ is alkyl C1 to 6,

or a pharmaceutically acceptable acid addition salt of those compounds containing a basic nitrogen atom, which comprises

a) production of a compound of formula I in which $R_3$ is $-CH(OR_4)_2$ or $-CF_3$ by

i) reaction of a compound of formula II,

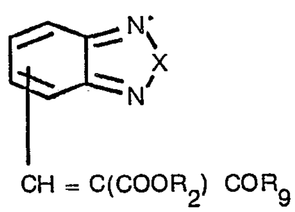

II

in which $R_9$ is a group $-CF_3$ or $-CH(OR_4)_2$, and X, $R_2$ and $R_4$ are as defined above, with a compound of formula III,

$$R_1OOCCH{=}C(R_8)NH_2 \qquad III$$

in which $R_1$ and $R_8$ are as defined above, or

ii) when $R_3$ in the product is to be $-CH(OR_4)_2$ reaction of a corresponding compound of formula I in which $R_3$ is $-CHO$ with an alcohol $R_4OH$,

b) production of a compound of formula I in which $R_3$ is $-CHO$ by selective hydrolysis of a corresponding compound of formula I in which $R_3$ is $-CH(OR_4)_2$,

c) production of a compound of formula I in which $R_3$ is $-CH{=}NOH$ by reaction of a corresponding compound of formula I in which $R_3$ is $-CHO$ with hydroxylamine, or a salt thereof,

d) production of a compound of formula I in which $R_3$ is $-CN$ by dehydration of a corresponding compound of formula I in which $R_3$ is $-CH{=}NOH$,

e) production of a compound of formula I in which $R_3$ is $-CH_2OH$ by selective reduction of a corresponding compound of formula I in which $R_3$ is $-CHO$,

f) production of a compound of formula I in which $R_2$ is hydrogen, by selective hydrolysis of a corresponding compound of formula I in which $-COOR_2$ is an ester group,

g) production of a compound of formula I in which $R_2$ is other than hydrogen by esterification, with a compound $R_2L$ in which L is a leaving group, of a corresponding compound of formula I in which $R_2$ is hydrogen, or $R_2$ together with $R_3$ forms a chain $-CHOH-$, or

h) selective reduction of a corresponding pyridine,

and where desired or necessary converting the resulting compound of formula I to a pharmaceutically acceptable acid addition salt thereof, or vice versa.

2. A process according to Claim 1 in which X is oxygen and the benzofurazanyl group is attached through its 4-position to the tetrahydropyridine ring.

3. A process according to Claim 1 or 2, wherein $R_1$ and $R_2$ are selected from ethyl and isopropyl.

4. A process according to any one of the preceding claims, wherein $R_3$ is —$CH_2OH$, —CHO or —CN.

5. A process according to Claim 4, wherein $R_3$ is —CHO or —CN.

6. A process according to any one of the preceding claims, wherein $R_8$ is methyl.

7. A process according to Claim 1, wherein the benzofurazanyl or benzothiadiazolyl group is attached to the dihydropyridine moiety through its 4-position,

$R_1$ and $R_2$, which may be the same or different, each represent alkyl C1 to 6, and

$R_3$ represents —$CH_2OH$, —CN, —$CH(OR_4)_2$, —CHO or —CH=NOH, and

$R_8$ is methyl.

8. A process according to Claim 1, wherein the benzofurazanyl or benzothiadiazolyl group is attached to the dihydropyridine moiety through its 4-position,

$R_3$ represent —$CH_2OH$, —CN, —$CH(OR_4)_2$, —CHO, —CH=NOH, or, together with $R_2$ forms a chain —CHOH—, and

$R_8$ is methyl,

and pharmaceutically acceptable salts of those compounds containing a group ——$(CH_2)_nNR_6R_7$.

9. A process according to Claim 7, wherein X is oxygen, and $R_3$ is —CN, —CH=NOH, —CHO or —$CH(OR_4)_2$.

10. A process according to Claim 7, wherein X is oxygen, and $R_3$ is —CN.

11. A process according to Claim 7, wherein X is oxygen, $R_3$ is —CN, one of $R_1$ and $R_2$ is methyl and the other is isopropyl.

12. A process according to Claim 1, wherein the compound of formula I is diethyl 4-(4-benzofurazanyl)-2-(diethoxymethyl)-1,4-dihydro-6-methylpyridine-3,5-dicarboxylate,

Dimethyl 4-(4-benzofurazanyl)-1,4-dihydro-2-(dimethoxymethyl)-5-methylpyridine-3,5-dicarboxylate,

3-Ethyl 5-(1-methylethyl)-4-(4-benzofurazanyl)-2-(diethoxymethyl)-1,4-dihydro-6-methylpyridine-3,5-dicarboxylate,

Diethyl 4-[4-(2,1,3-benzothiadiazolyl)]-2-(diethoxymethyl)-1,4-dihydro-6-methylpyridine-3,5-dicarboxylate,

Diethyl 4-(4-benzofurazanyl)-1,4-dihydro-2-formyl-6-methylpyridine-3,5-dicarboxylate,

Dimethyl 4-(4-benzofurazanyl)-1,4-dihydro-2-formyl-6-methylpyridine-3,5-dicarboxylate,

3-Ethyl 5-(1-methylethyl) 4-(4-benzofurazanyl)-1,4-dihydro-2-formyl-6-methylpyridine-3,5-dicarboxylate,

Diethyl 4-[4-(2,1,3-benzothiadiazolyl)]-1,4-dihydro-2-formyl-6-methylpyridine-3,5-dicarboxylate,

3-Ethyl 5-(2-methylpropyl) 4-(4-benzofurazany)1,4-dihydro-2-formyl-6-methylpyridine-3,5-dicarboxylate,

3-Ethyl 5-(2-(N-methyl-N-(phenylmethyl)amino)ethyl)4-(4-benzofurazanyl)-1,4-dihydro-2-formyl-6-methylpyridine-3,5-dicarboxylate hydrochloride,

3-Ethyl 5-(1-methylethyl) 4-(4-benzofurazanyl)-1,4-dihydro-6-methyl-2-[bis(1-methylethoxy)-methyl]pyridine-3,5-dicarboxylate,

4-(4-Benzofurazanyl)-1,4-dihydro-2-formyl-6-methylpyridine-3,5-dicarboxylic acid, 5-methyl ester,

Methyl 4-(4-benzofurazanyl)-1,4,5,7-tetrahydro-7-hydroxy 5-oxo-2-methylfuro[3,4-b]pyridine-3-carboxylate,

5-Methyl 3-(1-methylethyl) 4-(4-benzofurazanyl)-1,4-dihydro-2-formyl-6-methylpyridine-3,5-dicarboxylate,

5-Methyl 3-(2-phenoxyethyl) 4-(4-benzofurazanyl)-1,4-dihydro-2-formyl-6-methylpyridine-3,5-dicarboxylate,

3-(2-Ethoxyethyl) 5-methyl 4-(4-benzofurazanyl)-1,4-dihydro-2-formyl-6-methylpyridine-3,5-dicarboxylate,

3-(2-(N,N-diethylamino)ethyl) 5-methyl 4-(4-benzofurazanyl)-1,4-dihydro-2-formyl-6-methylpyridine-3,5-dicarboxylate hydrochloride,

Diethyl 4-(4-benzofurazanyl)-1,4-dihydro-2-(hydroxymethyl)-6-methylpyridine-3,5-dicarboxylate,

Dimethyl 4-(4-benzofurazanyl)-1,4-dihydro-2-(hydroxymethyl)-6-methylpyridine-3,5-dicarboxylate,

3-Ethyl 5-(1-methylethyl) 4-(4-benzofurazanyl)-1,4-dihydro-2-(hydroxymethyl)-6-methylpyridine-3,5-dicarboxylate,

Diethyl 4-[4-(2,1,3-benzothiadiazolyl)]-1,4-dihydro-2-(hydroxymethyl)-6-methylpyridine-3,5-dicarboxylate,

3-Ethyl 5-(2-methylpropyl) 4-(4-benzofurazanyl)-1,4-dihydro-2-(hydroxymethyl)-6-methylpyridine-3,5-dicarboxylate,

Diethyl 4-(4-benzofurazanyl)-2-cyano-1,4-dihydro-6-methylpyridine-3,5-dicarboxylate,

Dimethyl 4-(4-benzofurazanyl)-2-cyano-1,4-dihydro-6-methylpyridine-3,5-dicarboxylate,

3-Ethyl 5-(1-methylethyl) 4-(4-benzofurazanyl)-2-cyano-1,4-dihydro-6-methylpyridine-3,5-dicarboxylate,

Diethyl 4-[4-(2,1,3-benzothiadiazolyl)]-2-cyano-1,4-dihydro-6-methylpyridine-3,5-dicarboxylate,

3-Ethyl 5-(2-methylpropyl) 4-(4-benzofurazanyl)-2-cyano-1,4-dihydro-6-methylpyridine-3,5-dicarboxylate,

3-Ethyl 5-(2-methylpropyl) 4-(4-benzofurazanyl)-1,4-dihydro-2-(hydroxyiminomethyl)-6-methylpyridine-3,5-dicarboxylate, or

Diethyl 4-(4-benzofurazanyl)-1,4-dihydro-2-methyl-6-(trifluoromethyl)-pyridine-3,5-dicarboxylate.

**Patentansprüche für die Vertragsstaaten: BE CH DE FR GB IT LI LU NL SE**

1. Eine Verbindung der Formel (I)

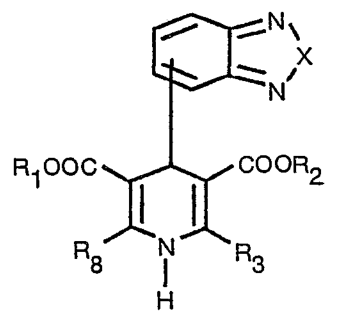

I

worin X Sauerstoff oder Schwefel ist; $R_1$ und $R_2$, die gleich oder verschieden sein können, jeweils $C_{1-6}$-Alkyl, $—(CH_2)_nOR_5$ oder $—(CH_2)_nNR_6R_7$ bedeuten und $R_2$ außerdem noch Wasserstoff darstellen kann; n eine ganze Zahl von 2 bis inkl. 4 ist; $R_3$ $—CH_2OH$, —CN, $—CH(OR_4)_2$, —CHO, —CH=NOH oder $—CF_3$ darstellt oder zusammen mit $R_2$ eine Kette —CHOH— bildet; $R_4$ $C_{1-6}$-Alkyl ist; $R_5$ $C_{1-6}$-Alkyl oder Phenyl bedeutet; $R_6$ und $R_7$, die gleich oder verschieden sein können, jeweils $C_{1-6}$-Alkyl gegebenenfalls substituiert durch Phenyl darstellen; und $R_8$ $C_{1-6}$-Alkyl ist, sowie pharmazeutisch annehmbare Säureadditionssalze jener Verbindungen, die ein basisches Stickstoffatom enthalten.

2. Verbindung gemäß Anspruch 1, worin X Sauerstoff ist und die Benzofurazanylgruppe über die 4-Stellung an den Tetrahydropyridinring gebunden ist.

3. Verbindung gemäß Anspruch 1 oder 2, worin $R_1$ und $R_2$ aus Äthyl und Isopropyl ausgewählt sind.

4. Verbindung gemäß einem der vorhergehenden Ansprüche, worin $R_3$ $—CH_2OH$, —CHO oder —CN ist.

5. Verbindung gemäß Anspruch 4, worin $R_3$ —CHO oder —CN ist.

6. Verbindung gemäß einem der vorhergehenden Ansprüche, worin $R_8$ Methyl ist.

7. Verbindung gemäß Anspruch 1, worin die Benzofurazanyl-oder Benzothiadiazolylgruppe über die 4-Stellung an die Dihydropyridingruppe gebunden ist; $R_1$ und $R_2$, die gleich oder verschieden sein können, jeweils $C_{1-6}$-Alkyl bedeuten; $R_3$ $—CH_2OH$, —CN, $—CH(OR_4)_2$, —CHO oder —CH=NOH ist und $R_8$ Methyl darstellt.

8. Verbindung gemäß Anspruch 1, worin die Benzofurazanyl-oder Benzothiadiazolylgruppe über die 4-Stellung an die Dihydropyridingruppe gebunden ist; $R_3$ $—CH_2OH$, —CN, $—CH(OR_4)_2$, —CHO oder CH=NOH bedeutet oder zusammen mit $R_2$ eine Kette —CHOH bildet, und $R_8$ Methyl ist, und pharmazeutisch annehmbare Salze jener Verbindungen, die eine Gruppe $—(CH_2)_nNR_6R_7$ enthalten.

9. Verbindung gemäß Anspruch 7, worin X Sauerstoff und $R_3$ —CN, —CH=NOH, —CHO oder $—CH(OR_4)_2$ bedeuten.

10. Verbindung gemäß Anspruch 7, worin X Sauerstoff und $R_3$ —CN bedeuten.

11. Verbindung gemäß Anspruch 7, worin X Sauerstoff, $R_3$ —CN, eines von $R_1$ und $R_2$ Methyl und das andere Isopropyl bedeuten.

12. Diäthyl-4-(4-benzofurazanyl)-2-(diäthoxymethyl)-1,4-dihydro-6-methylpyridin-3,5-dicarboxylat,

Dimethyl-4-(4-benzofurazanyl)-1,4-dihydro-2-(dimethoxymethyl)-6-methylpyridin-3,5-dicarboxylat,

3-Äthyl-5-(1-methyläthyl)-4-(4-benzofurazanyl)-2-(diäthoxymethyl)-1,4-dihydro-6-methylpyridin-3,5-dicarboxylat,

Diäthyl-4-[4-(2,1,3-benzothiadiazolyl)]-2-(diäthoxymethyl)-1,4-dihydro-6-methylpyridin-3,5-dicarboxylat,

Diäthyl-4-(4-benzofurazanyl)-1,4-dihydro-2-formyl-6-methylpyridin-3,5-dicarboxylat,

Dimethyl-4-(4-benzofurazanyl)-1,4-dihydro-2-formyl-6-methylpyridin-3,5-dicarboxylat,

3-Äthyl-5-(1-methyläthyl)-4-(4-benzofurazanyl)-1,4-dihydro-2-formyl-6-methylpyridin-3,5-dicarboxylat,

Diäthyl-4-[4-(2,1,3-benzothiadiazolyl)]-1,4-dihydro-2-formyl-6-methylpyridin-3,5-dicarboxylat,

3-Äthyl-5-(2-methylpropyl)-4-(4-benzofurazanyl)-1,4-dihydro-2-formyl-6-methylpyridin-3,5-dicarboxylat,

3-Äthyl-5-(2-(N-methyl-N-(phenylmethyl)-amino)-äthyl)-4-(4-benzofurazanyl)-1,4-dihydro-2-formyl-6-methylpyridin-3,5-dicarboxylat-hydrochlorid,

3-Äthyl-5-(1-methyläthyl)-4-(4-benzofurazanyl)-1,4-dihydro-6-methyl-2-[bis-(1-methyläthoxy)-methyl]-pyridin-3,5-dicarboxylat,

4-(4-Benzofurazanyl)-1,4-dihydro-2-formyl-6-methylpyridin-3,5-dicarbonsäure-5-methylester,

Methyl-4-(4-benzofurazanyl)-1,4,65,7-tetrahydro-7-hydroxy-5-oxo-2-methylfuro[3,4-b]pyridin-3-carboxylat,

5-Methyl-3-(1-methyläthyl-4-(4-benzofurazanyl)-1,4-dihydro-2-formyl-6-methylpyridin-3,5-dicarboxylat,

5-Methyl-3-(2-phenoxyäthyl)-4-(4-benzofurazanyl)-1,4-dihydro-2-formyl-6-methylpyridin-3,5-dicarboxylat,

3-(2-Äthoxyäthyl)-5-methyl-4-(4-benzofurazanyl)-1,4-dihydro-2-formyl-6-methylpyridin-3,5-dicarboxylat,

3-(2-(N,N-Diäthylamino)-äthyl)-5-methyl-4-(4-benzofurazanyl)-1,4-dihydro-2-formyl-6-methylpyridin-3,5-dicarboxylat-hydrochlorid,

Diäthyl-4-(4-benzofurazanyl)-1,4-dihydro-2-(hydroxymethyl)-6-methylpyridin-3,5-dicarboxylat,

Dimethyl-4-(4-benzofurazanyl)-1,4-dihydro-2-(hydroxymethyl)-6-methylpyridin-3,5-dicarboxylat,

3-Äthyl-5-(1-methyläthyl)-4-(4-benzofurazanyl)-1,4-dihydro-2-(hydroxymethyl)-6-methylpyridin-3,5-dicarboxylat,

Diäthyl-4-[4-(2,1,3-benzothiadiazolyl]-1,4-dihydro-2-(hydroxymethyl)-6-methylpyridin-3,5-dicarboxylat,

3-Äthyl-5-(2-methylpropyl-4-(4-benzofurazanyl)-1,4-dihydro-2-(hydroxymethyl)-6-methylpyridin-3,5-dicarboxylat,

Diäthyl-4-(4-benzofurazanyl)-2-cyano-1,4-dihydro-6-methylpyridin-3,5-dicarboxylat,

Dimethyl-4-(4-benzofurazanyl)-2-cyano-1,4-dihydro-6-methylpyridin-3,5-dicarboxylat,

3-Äthyl-5-(1-methyläthyl)-4-benzofurazanyl)-2-cyano-1,4-dihydro-6-methylpyridin-3,5-dicarboxylat,

Diäthyl-4-[4-(2,1,3-benzothiadiazolyl)]-2-cyano-1,4-dihydro-6-methylpyridin-3,5-dicarboxylat,

3-Äthyl-5-(2-methylpropyl)-4-(4-benzofurazanyl)-2-cyano-1,4-dihydro-6-methylpyridin-3,5-dicarboxylat,

3-Äthyl-5-(2-methylpropyl)-4-(4-benzofurazanyl)-1,4-dihydro-2-(hydroxyiminomethyl)-6-methylpyridin-3,5-dicarboxylat oder

Diäthyl-4-(4-benzofurazanyl)-1,4-dihydro-2-methyl-6-(trifluormethyl)-pyridin-3,5-dicarboxylat.

13. Pharmazeutische Formulierung, die eine Verbindung gemäß einem der vorhergehenden Ansprüche in Mischung mit einem pharmazeutisch annehmbaren Adjuvans, Verdünnungsmittel oder Träger aufweist.

14. Verfahren zum Herstellen einer Verbindung der Formel (I), wie in Anspruch 1 definiert, oder eines pharmazeutisch annehmbaren Säureadditionssalzes hievon, welches umfaßt:

a) die Herstellung einer Verbindung der Formel (I), worin $R_3$ —$CH(OR_4)_2$ oder $CF_3$ bedeutet, durch

i) Umsetzen einer Verbindung der Formel (II)

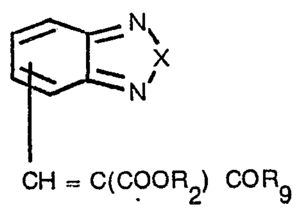

II

worin $R_9$ eine Gruppe —$CF_3$ oder —$CH(OR_4)_2$ bedeutet und X, $R_2$ und $R_4$ wie oben definiert sind, mit einer Verbindung der Formel (III)

$$R_1OOCCH=C(R_8)NH_2 \qquad (III),$$

worin $R_1$ und $R_8$ wie oben definiert sind, oder

ii) wenn $R_3$ im Produkt $CH(OR_4)_2$ sein soll, Umsetzen einer entsprechenden Verbindung der Formel (I), worin $R_3$ —CHO ist, mit einem Alkohol $R_4OH$.

b) die Herstellung einer Verbindung der Formel (I), worin $R_3$ —CHO ist, durch selektive Hydrolyse einer entsprechenden Verbindung der Formel (I), worin $R_3$ —$CH(OR_4)_2$ ist,

c) die Herstellunbg einer Verbindung der Formel (I), worin $R_3$ —CH=NOH bedeutet, durch Umsetzen einer entsprechenden Verbindung der Formel (I), worin $R_3$ —CHO ist, mit Hydroxylamin oder einem Salz hievon,

d) die Herstellung einer Verbindung der Formel (I), worin $R_3$ —CN bedeutet, durch Dehydratisieren einer entsprechenden Verbindung der Formel (I), worin $R_3$ —CH=NOH ist,

e) die Herstellung einer Verbindung der Formel (I), worin $R_3$ —$CH_2OH$ ist, durch selektives Reduzieren einer entsprechenden Verbindung der Formel (I), worin $R_3$ —CHO ist,

f) die Herstellung einer Verbindung der Formel (I), worin $R_2$ Wasserstoff ist, durch selektive Hydrolyse einer entsprechenden Verbindung der Formel (I), worin $—COOR_2$ eine Estergruppe bedeutet,

g) die Herstellung einer Verbindung der Formel (I), worin $R_2$ von Wasserstoff verschieden ist, durch Verestern einer entsprechenden Verbindung der Formel (I), worin $R_2$ Wasserstoff ist oder $R_2$ zusammen mit $R_3$ eine Kette —CHOH— bildet, mit einer Verbindung $R_2L$, worin L eine abspaltbare gruppe ist, oder

h) die selektive Reduktion eines entsprechenden Pyridins und, wenn gewünscht oder notwendig, Überführen der resultierenden Verbindung der Formel (I) in ein pharmazeutisch annehmbares Säureadditionssalz hievon oder vice versa.

15. Verbindung gemäß einem der Ansprüche 1 bis 12 zur Verwendung als Pharmazeutikum.

16. Verwendung einer Verbindung gemäß einem der Ansprüche 1 bis 12 zur Herstellung eines Medikaments für die Behandlung von Bluthochdruck, Angina pectoris, Variant Angina, kongestiver Herzlähmung, Myocardinfarkt, cerebral-vaskulärer Störung, vaskulärerErkrankung oder Asthma.

**Patentansprüche für den Vertragsstaat: AT**

1. Verfahren zur Herstellung einer Verbindung der Formel

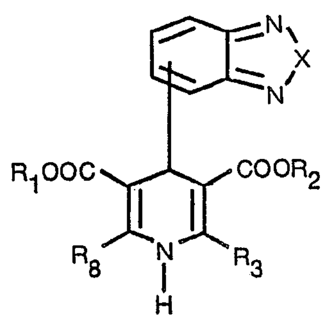

I

worin X Sauerstoff oder Schwefel ist; $R_1$ und $R_2$, die gleich oder verschieden sein können, jeweils $C_{1-6}$-Alkyl $—(CH_2)_nOR_5$ oder $—(CH_2)_nNR_6R_7$ bedeuten und $R_2$ außerdem noch Wasserstoff darstellen kann; n eine ganze Zahl von 2 bis inkl. 4 ist; $R_3$ $—CH_2OH$, —CN, $—CH(OR_4)_2$, —CHO, —CH=NOH oder $—CF_3$ darstellt oder zusammen mit $R_2$ eine Kette —CHOH— bildet; $R_4$ $C_{1-6}$-Alkyl ist; $R_5$ $C_{1-6}$-Alkyl oder Phenyl bedeutet; $R_6$ und $R_7$, die gleich oder verschieden sein können, jeweils $C_{1-6}$-Alkyl gegebenenfalls substituiert durch Phenyl darstellen; und $R_8$ $C_{1-6}$-Alkyl ist, sowie pharmazeutisch annehmbaren Säureadditionssalzen jener Verbindungen, die ein basisches Stickstoffatom enthalten, welches umfaßt:

a) die Herstellung einer Verbindung der Formel (I), worin $R_3$ $—CH(OR_4)_2$ oder $CF_3$ bedeutet, durch

i) Umsetzen einer Verbindung der Formel (II)

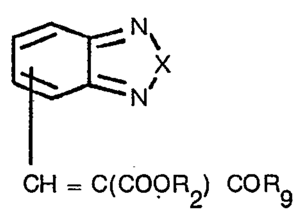

II

worin $R_9$ eine Gruppe $—CF_3$ oder $—CH(OR_4)_2$ bedeutet und X, $R_2$ und $R_4$ wie oben definiert sind, mit einer Verbindung der Formel (III)

$$R_1OOCCH=C(R_8)NH_2 \qquad \text{(III)},$$

worin $R_1$ und $R_8$ wie oben definiert sind, oder

ii) wenn $R_3$ im Produkt $CH(OR_4)_2$ sein soll, Umsetzen einer entsprechenden Verbindung der Formel (I), worin $R_3$ —CHO ist, mit einem Alkohol $R_4OH$,

b) die Herstellung einer Verbindung der Formel (I), worin $R_3$ —CHO ist, durch selektive Hydrolyse einer entsprechenden Verbindung der Formel (I), worin $R_3$ $—CH(OR_4)_2$ ist,

c) die Herstellung einer Verbindung der Formel (I), worin $R_3$ —CH=NOH bedeutet, durch Umsetzen einer entsprechenden Verbindung der Formel (I), worin $R_3$ —CHO ist, mit Hydroxylamin oder einem Salz hievon,

d) die Herstellung einer Verbindung der Formel (I), worin $R_3$ —CN bedeutet, durch Dehydratisieren einer entsprechenden Verbindung der Formel (I), worin $R_3$ —CH=NOH ist,

e) die Herstellung einer Verbindung der Formel (I), worin $R_3$ —$CH_2OH$ ist, durch selektives Reduzieren einer entsprechenden Verbindung der Formel (I), worin $R_3$ —CHO ist,

f) die Herstellung einer Verbindung der Formel (I), worin $R_2$ Wasserstoff ist, durch selektive Hydrolyse einer entsprechenden Verbindung der Formel (I), worin —$COOR_2$ eine Estergruppe bedeutet,

g) die Herstellung einer Verbindung der Formel (I), worin $R_2$ von Wasserstoff verschieden ist, durch Verestern einer entsprechenden Verbindung der Formel (I), worin $R_2$ Wasserstoff ist oder $R_2$ zusammen mit $R_3$ eine Kette —CHOH— bildet, mit einer Verbindung $R_2L$, worin L eine abspaltbare Gruppe ist, oder

h) die selektive Reduktion eines entsprechenden Pyridins und, wenn gewünscht oder notwendig, Überführen der resultierenden Verbindung der Formel (I) in ein pharmazeutisch annehmbares Säureadditionssalz hievon oder vice versa.

2. Verfahren gemäß Anspruch 1, worin X Sauerstoff ist und die Benzofurazanylgruppe über die 4-Stellung an den Tetrahydropyridinring gebunden ist.

3. Verfahren gemäß Anspruch 1 oder 2, worin $R_1$ und $R_2$ aus Äthyl und Isopropyl ausgewählt sind.

4. Verfahren gemäß einem der vorhergehenden Ansprüche, worin $R_3$ —$CH_2OH$, —CHO oder —CN ist.

5. Verfahren gemäß Anspruch 4, worin $R_3$ —CHO oder —CN ist.

6. Verfahren gemäß einem der vorhergehenden Ansprüche, worin $R_8$ Methyl ist.

7. Verfahren gemäß Anspruch 1, worin die Benzofurazanyl-oder Benzothiadiazolylgruppe über die 4-Stellung an die Dihydropyridingruppe gebunden ist; $R_1$ und $R_2$, die gleich oder verschieden sein können, jeweils $C_{1-6}$-Alkyl bedeuten; $R_3$ —$CH_2OH$, —CN, —$CH(OR_4)_2$, —CHO oder —CH=NOH ist und $R_8$ Methyl darstellt.

8. Verfahren gemäß Anspruch 1, worin die Benzofurazanyl-oder Benzothiadiazolylgruppe über die 4-Stellung an die Dihydropyridingruppe gebunden ist; $R_3$ —$CH_2OH$, —CN, —$CH(OR_4)_2$, —CHO oder CH=NOH bedeutet oder zusammen mit $R_2$ eine Kette —CHOH bildet, und $R_8$ Methyl ist, und pharmazeutisch annehmbare Salze jener Verbindungen, die eine Gruppe —$(CH_2)_nNR_6R_7$ enthalten.

9. Verfahren gemäß Anspruch 7, worin X Sauerstoff und $R_3$ —CN, —CH=NOH, —CHO oder —$CH(OR_4)_2$ bedeuten.

10. Verfahren gemäß Anspruch 7, worin X Sauerstoff und $R_3$ —CN bedeuten.

11. Verfahren gemäß Anspruch 7, worin X Sauerstoff, $R_3$ —CN, eines von $R_1$ und $R_2$ Methyl und das andere Isopropyl bedeuten.

12. Verfahren gemäß Anspruch 1, worin die Verbindung der Formel (I) ist:

Diäthyl-4-(4-benzofurazanyl)-2-(diäthoxymethyl)-1,4-dihydro-6-methylpyridin-3,5-dicarboxylat,

Dimethyl-4-(4-benzofurazanyl)-1,4-dihydro-2-(dimethyoxymethyl)-6-methylpyridin-3,5-dicarboxylat,

3-Äthyl-5-(1-methyläthyl)-4-(4-benzofurazanyl)-2-(diäthoxymethyl)-1,4-dihydro-6-methylpyridin-3,5-dicarboxylat,

Diäthyl-4[4-(2,1,3-benzothiadiazolyl)]-2-(diäthoxymethyl)-1,4-dihydro-6-methylpyridin-3,5-dicarboxylat,

Diäthyl-4-(4-benzofurazanyl)-1,4-dihydro-2-formyl-6-methylpyridin-3,5-dicarboxylat,

Dimethyl-4-(4-benzofurazanyl)-1,4-dihydro-2-formyl-6-methylpyridin-3,5-dicarboxylat,

3-Äthyl-5-(1-methyläthyl)-4-(4-benzofurazanyl)-1,4-dihydro-2-formyl-6-methylpyridin-3,5-dicarboxylat,

Diäthyl-4-[4-(2,1,3-benzothiadiazolyl)]-1,4-dihydro-2-formyl-6-methylpyridin-3,5-dicarboxylat,

3-Äthyl-5-(2-methylpropyl)-4-(4-benzofurazanyl)-1,4-dihydro-2-formyl-6-methylpyridin-3,5-dicarboxylat,

3-Äthyl-5-(2-N-methyl-N-(phenylmethyl)-amino)-äthyl)-4-(4-benzofurazanyl)-1,4-dihydro-2-formyl-6-methylpyridin-3,5-dicarboxylat-hydrochlorid,

3-Äthyl-5-(1-methyläthyl)-4-(4-benzofurazanyl)-1,4-dihydro-6-methyl-2-[bis-(1-methyläthoxy)-methyl]-pyridin-3,5-dicarboxylat,

4-(4-Benzofurazanyl)-1,4-dihydro-2-formyl-6-methylpyridin-3,5-dicarbonsäure-5-methylester,

Methyl-4-(4-benzofurazanyl)-1,4,5,7-tetrahydro-7-hydroxy-5-oxo-2-methylfuro[3,4-b]pyridin-3-carboxylat,

5-Methyl-3-(1-methyläthyl-4-(4-benzofurazanyl)-1,4-dihydro-2-formyl-6-methylpyridin-3,5-dicarboxylat,

5-Methyl-3-(2-phenoxyäthyl)-4-(4-benzofurazanyl)-1,4-dihydro-2-formyl-6-methylpyridin-3,5-dicarboxylat,

3-(2-Äthoxyäthyl)-5-methyl-4-(4-benzofurazanyl)-1,4-dihydro-2-formyl-6-methylpyridin-3,5-dicarboxylat,

3-(2-(N,N-Diäthylamino)-äthyl)-5-methyl-4-(4-benzofurazanyl)-1,4-dihydro-2-formyl-6-methylpyridin-3,5-dicarboxylat-hydrochlorid,

Diäthyl-4-(4-benzofurazanyl)-1,4-dihydro-2-(hydroxymethyl)-6-methylpyridin-3,5-dicarboxylat,

Dimethyl-4-(4-benzofurazanyl)-1,4-dihydro-2-(hydroxymethyl)-6-methylpyridin-3,5-dicarboxylat,

3-Äthyl-5-(1-methyläthyl)-4-(4-benzofurazanyl)-1,4-dihydro-2-(hydroxymethyl)-6-methylpyridin-3,5-dicarboxylat,

Diäthyl-4-[4-(2,1,3-benzothiadiazolyl)]-1,4-dihydro-2-(hydroxymethyl)-6-methylpyridin-3,5-dicarboxylat,

3-Äthyl-5-(2-methylpropyl-4-(4-benzofurazanyl)-1,4-dihydro-2-(hydroxymethyl)-6-methylpyridin-3,5-dicarboxylat,

Diäthyl-4-(4-benzofurazanyl)-2-cyano-1,4-dihydro-6-methylpyridin-3,5-dicarboxylat,

Dimethyl-4-(4-benzofurazanyl)-2-cyano-1,4-dihydro-6-methylpyridin-3,5-dicarboxylat,

3-Äthyl-5-(1-methyläthyl)-4-(4-benzofurazanyl)-2-cyano-1,4-dihydro-6-methylpyridin-3,5-dicarboxylat,

Diäthyl-4-[4-(2,1,3-benzothiadiazolyl)]-2-cyano-1,4-dihydro-6-methylpyridin,3,5-dicarboxylat,

3-Äthyl-5-(2-methylpropyl)-4-(4-benzofurazanyl)-2-cyano-1,4-dihydro-6-methylpyridin-3,5-dicarboxylat,

3-Äthyl-5-(2-methylpropyl)-4-(4-benzofurazanyl)-1,4-dihydro-2-(hydroxyiminomethyl)-6-methylpyridin-3,5-dicarboxylat oder

Diäthyl-4-(4-benzofurazanyl)-1,4-dihydro-2-methyl-6-(trifluormethyl)-pyridin-3,5-dicarboxylat.

**Revendications pour les Etats contractants: BE CH DE FR GB IT LI LU NL SE**

1. Composé de formule I:

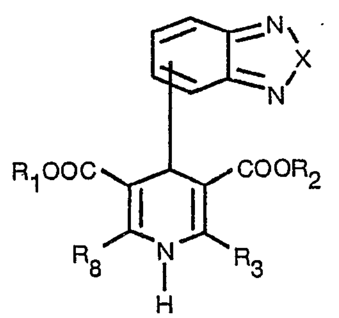

I

où X représente un atome d'oxygène ou de soufre,

$R_1$ et $R_2$, qui peuvent être identiques ou différents, représentent chacun un radical alcoyle en C1 à 6, $—(CH_2)_nOR_5$ ou $—(CH_2)_nNR_6R_7$, et en outre $R_2$ peut représenter un atome d'hydrogène,

n représente un nombre entier de 2 à 4 inclusivement,

$R_3$ représente un radical $—CH_2OH$, —CN, $—CH(OR_4)_2$, —CHO, —CH=NOH, $—CF_3$, ou, conjointement avec $R_2$, forme une chaîne —CHOH—,

$R_4$ représente un radical alcoyle en C1 à 6,

$R_5$ représente un radical alcoyle en C 1 à 6 ou phényle,

$R_6$ et $R_7$, qui peuvent être identiques ou différents, représentent chacun un radical alcoyle en C 1 à 6 éventuellement substitué par phényle, et

$R_8$ représente un radical alcoyle en C 1 à 6, et les sels d'addition d'acides pharmaceutiquement acceptables de ceux de ces composés qui contiennent un atome d'azone basique.

2. Composé suivant la revendication 1, dans lequel X représente un atome d'oxygène et le radical benzofurazanyle est uni par sa position 4 au cycle tétrahydropyridine,

3. Composé suivant la revendication 1 ou 2, dans lequel $R_1$ et $R_2$ sont choisis parmi les radicaux éthyle et isopropyle.

4. Composé suivant l'une quelconque des revendications précédentes, dans lequel $R_3$ représente un radical $—CH_2OH$, —CHO ou —CN.

5. Composé suivant la revendication 4, dans lequel $R_3$ représente un radical —CHO ou —CN.

6. Composé suivant l'une quelconque des revendications précédentes, dans lequel $R_8$ représente un radical méthyle.

7. Composé suivant la revendication 1, dans lequel le radical benzofurazanyle ou benzothiadiazolyle est uni à l'entité dihydropyridine par sa position 4,

$R_1$ et $R_2$, qui peuvent être identiques ou différents, représentent chacun un radical alcoyle en C 1 à 6, et

$R_3$ représente un radical $—CH_2OH$, —CN, $—CH(OR_4)_2$, —CHO ou —CH=NOH, et

$R_8$ représente un radical méthyle.

8. Composé suivant la revendication 1, dans lequel le radical benzofurazanyle ou benzothiodiazolyle est uni à l'entité dihydropyridine par sa position 4,

$R_3$ représente un radical $—CH_2OH$, —CN, $—CH(OR_4)_2$, —CHO, —CH=NOH, ou, conjointement avec R2, forme une chaîne, —CHOH—, et

$R_8$ représente un radical méthyle,

et les sels pharmaceutiquement acceptables de ceux de ces composés qui contiennent un radical $—(CH_2)_nNR_6R_7$.

9. Composé suivant la revendication 7, dans lequel X représente un atome d'oxygène, et

$R_3$ représente un radical —CN, —CH=NOH, —CHO ou $—CH(OR_4)_2$.

10. Composé suivant la revendication 7, dans lequel X représente un atome d'oxygène, et $R_3$ représente le radical —CN.

11. Composé suivant la revendication 7, dans lequel X représente un atome d'oxygène, $R_3$ représente un radical —CN, l'un d'entre $R_1$ et $R_2$ représente un radical méthyle, et l'autre représente un radical isopropyle.

12. Le 4-(4-benzofurazanyl)-2-(diéthoxyméthyl)-1,4-dihydro-6-méthylpyridine-3,5-dicarboxylate de diéthyle,

le 4-(4-benzofurazanyl)-1,4-dihydro-2-(diméthoxyméthyl)-6-méthylpyridine-3,5-dicarboxylate de diméthyle,

le 4-(4-benzofurazanyl)-2-(diéthoxyméthyl)-1-4-dihydro-6-méthylpyridine-3,5-dicarboxylate de 3-éthyle et de 5-(1-méthyléthyle),

le 4-[4-(2,1,3-benzothiadiazolyl)]-2-(diéthoxyméthyl)-1,4-dihydro-6-méthylpyridine-3,5-dicarboxylate de diéthyle,

le 4-(4-benzofurazanyl)-1,4-dihydro-2-formyl-6-méthylpyridine-3,5-dicarboxylate de diéthyle,

le 4-(4-benzofurazanyl)-1,4-dihydro-2-formyl-6-méthylpyridine-3,5-dicarboxylate de diméthyle,

le 4-(4-benzofurazanyl)-1,4-dihydro-2-formyl-6-méthylpyridine-3,5-dicarboxylate de 3-éthyle et de 5-(1-méthyléthyle),

le 4-[4-(2,1,3-benzothiadiazolyl)]-1,4-dihydro-2-formyl-6-méthylopyridine-3,5-dicarboxylate de diéthyle,

le 4-(4-benzofurazanyl)-1,4-dihydro-2-formyl-6-méthylpyridine-3,5-dicarboxylate de 3-éthyle et de 5-(2-méthylpropyle),

le chlorhydrate de 4-(4-benzofurazanyl)-1,4-dihydro-2-formyl-6-méthylpyridine-3,5-dicarboxylate de 3-éthyle et de 5-(2-(N-méthyl-N-(phénylméthyl)amino)-éthyle,

le 4-(4-benzofurazanyl)-1,4-dihydro-6-méthyl-2-[bis(1-méthyléthoxy)-méthyl]pyridine-3,5-dicarboxylate de 3-éthyle et de 5-(1-méthyléthyle),

l'ester 5-méthylique d'acide 4-(4-benzofurazanyl)-1,4-dihydro-2-formyl-6-méthylpyridine-3,5-dicarboxylique,

le 4-(4-benzofurazanyl)-1,4,5,7-tétrahydro-7-hydroxy-5-oxo-2-méthylfuro[3,4-b]pyridine-3-carboxylate de méthyle,

le 4-(4-benzofurazanyl)-1,4-dihydro-2-formyl-6-méthylpyridine-3,5-dicarboxylate de 5-méthyle et de 3-(1-méthyléthyle),

le 4-(4-benzofurazanyl)-1,4-dihydro-2-formyl-6-méthylpyridine-3,5-dicarboxylate de 5-méthyle et de 3-(2-phénoxyéthyle),

le 4-(4-benzofurazanyl)-1,4-dihydro-2-formyl-6-méthylpyridine-3,5-dicarboxylate de 3-(2-éthoxyéthyle) et de 5-méthyle,

le chlorhydrate de 4-(4-benzofurazanyl)-1,4-dihydro-2-formyl-6-méthylpyridine-3,5-dicarboxylate de 3-(2-(N,N-diéthylamino)éthyle) et de 5-méthyle,

le 4-(4-benzofurazanyl)-1,4-dihydro-2-(hydroxyméthyl)-6-méthylpyridine-3,5-dicarboxylate de diéthyle,

le 4-(4-benzofurazanyl)-1,4-dihydro-2-(hydroxyméthyl)-6-méthylpyridine-3,5-dicarboxylate de diméthyle,

le 4-(4-benzofurazanyl)-1,4-dihydro-2-(hydroxyméthyl)-6-méthylpyridine-3,5-dicarboxylate de 3-éthyle et de 5-(1-méthyléthyle),

le 4-[4-(2,1,3-benzothiadiazolyl)]-1,4-dihydro-2-(hydroxyméthyl)-6-méthylpyridine-3,5-dicarboxylate de diéthyle,

le 4-(4-benzofurazanyl)-1,4-dihydro-2-(hydroxyméthyl)-6-méthylpyridine-3,5-dicarboxylate de 3-éthyle et de 5-(2-méthylpropyle),

le 4-(4-benzofurazanyl)-2-cyano-1,4-dihydro-6-méthylpyridine-3,5-dicarboxylate de diéthyle

le 4-(4-benzofurazanyl)-2-cyano-1,4-dihydro-6-méthylpyridine-3,5-dicarboxylate de diméthyle,

le 4-(4-benzofurazanyl)-2-cyano-1,4-dihydro-6-méthylpyridine-3,5-dicarboxylate de 3-éthyle et de 5-(1-méthyléthyle),

le 4-[4-(2,1,3-benzothiadiazolyl)]-2-cyano-1,4-dihydro-6-méthylpyridine-3,5-dicarboxylate de diéthyle,

le 4-(4-benzofurazanyl)-2-cyano-1,4-dihydro-6-méthylpyridine-3,5-dicarboxylate de 3-éthyle et de 5-(2-méthylpropyle),

le 4-(4-benzofurazanyl)-1,4-dihydro-2-(hydroxyiminométhyl)-6-méthylpyridine-3,5-dicarboxylate de 3-éthyle et de 5-(2-méthylpropyle), ou

le 4-(4-benzofurazanyl)-1,4-dihydro-2-méthyl-6-(trifluorométhyl)-pyridine-3,5-dicarboxylate de diéthyle.

13. Formulation pharmaceutique comprenant un composé suivant l'une quelconque des revendications précédentes en mélange avec un adjuvant, diluant ou excipient pharmaceutiquement acceptable.

14. Procédé de préparation d'un composé de formule I tel que défini dans la revendication 1, ou d'un sel d'addition d'acide pharmaceutiquement acceptable de celui-ci, qui comprend

a) la préparation d'un composé de formule I où $R_3$ représente un radical $—CH(OR_4)_2$ ou $—CF_3$ par

i) réaction d'un composé de formule II:

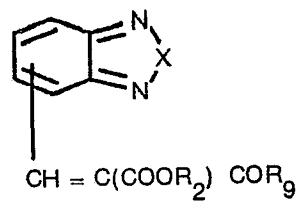

II

où $R_9$ représente un radical $—CF_3$ ou $—CH(OR_4)_2$, X, $R_2$ et $R_4$ sont tels que définis ci-dessus, avec un composé de formule III;

$$R_1OOCCH=C(R_8)NH_2 \quad (III)$$

où $R_1$ et $R_8$ sont tels que définis ci-dessus, ou

ii) lorsque $R_3$ dans le produit doit représenter un radical $—CH(OR_4)_2$, réaction d'un composé correspondant de formule I où $R_3$ représente un radical —CHO avec un alcool $R_4OH$,

b) la préparation d'un composé de formule I où $R_3$ représente un radical —CHO par hydrolyse sélective d'un composé correspondant de formule I où $R_3$ représente un radical $—CH(OR_4)_2$,

c) la préparation d'un composé de formule I où $R_3$ représente un radical —CH=NOH par réaction d'un composé correspondant de formule I où $R_3$ représente un radical —CHO avec l'hydroxylamine ou un sel de celle-ci,

d) la préparation d'un composé de formule I où $R_3$ représente un radical —CN par déshydratation d'un composé correspondant de formule I où $R_3$ représente un radical —CH=NOH,

e) la préparation d'un composé de formule I où $R_3$ représente un radical $—CH_2OH$ par réduction sélective d'un composé correspondant de formule I où $R_3$ représente un radical —CHO,

f) la préparation d'un composé de formule I où $R_2$ représente un atome d'hydrogène par hydrolyse sélective d'un composé correspondant de formule I où $—COOR_2$ représente un radical ester,

g) la préparation d'un composé de formule I où $R_2$ représente autre chose qu'un atome d'hydrogène par estérification, à l'aide d'un composé $R_2L$ où L représente un radical partant, d'un composé correspondant de formule I où $R_2$ représente un atome d'hydrogène ou bien $R_2$ conjointement avec $R_3$ forme une chaîne —CHOH—, ou

h) la réduction sélective d'une pyridine correspondante,

et, lorsque la chose est souhaitée ou nécessaire, la conversion du composé résultant de formule I en un sel d'addition d'acide pharmaceutiquement acceptable de celui-ci, et réciproquement.

15. Composé suivant l'une quelconque des revendications 1 à 12 utilisé comme agent pharmaceutique.

16. Utilisation d'un composé suivant l'une quelconque des revendications 1 à 12 pour la préparation d'un médicament destiné au traitement de l'hypertension, de l'angine de poitrine, de l'angine instable, de décompensation cardiaque congestive, de l'infarctus du myocarde, des troubles cérébro-vasculaires, d'une affection vasculaire ou de l'asthme.

**Revendications pour l'Etat contractant: AT**

1. Procédé de préparation d'un composé de formule I:

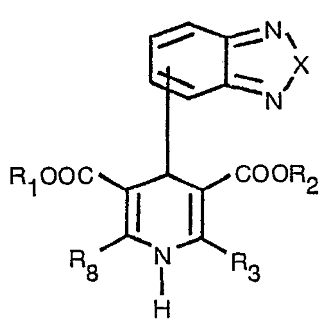

I

où X représente un atome d'oxygène ou de soufre,

$R_1$ et $R_2$, qui peuvent être identiques ou différents, représentent chacun un radical alcoyle en C 1 à 6, $—(CH_2)_nOR_5$ ou $—(CH_2)_nNR_6R_7$, et en outre $R_2$ peut représenter un atome d'hydrogène,

n représente un nombre entier de 2 à 4 inclusivement,

$R_3$ représente un radical $—CH_2OH$, —CN, $—(CH(OR_4)_2$, —CHO, —CH=NOH, $—CF_3$, ou, conjointement avec $R_2$, forme une chaîne —CHOH—,

$R_4$ représente un radical alcoyle en C 1 à 6,

$R_5$ représente un radical alcoyle en C 1 à 6 ou phényle,

$R_6$ et $R_7$, qui peuvent être identiques ou différents, représentent chacun un radical alcoyle en C 1 à 6 éventuellement substitué par phényle, et

$R_8$ représente un radical alcoyle en C 1 à 6, ou d'un sel d'addition d'acide pharmaceutiquement acceptable de deux de ces composés qui contiennent un atome d'azote basique, qui comprend

a) la préparation d'un composé de formule I où $R_3$ représente un radical $—CH(OR_4)_2$ ou $—CF_3$ par

i) réaction d'un composé de formule II:

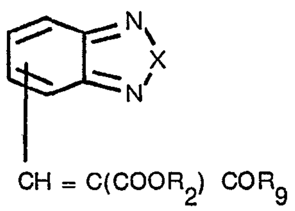

II

où $R_9$ représente un radical $—CF_3$ ou $—CH(OR_4)_2$, X, $R_2$ et $R_4$ sont tels que définis ci-dessus, avec un composé de formule III:

$$R_1OOCCH{=}C(R_8)NH_2 \qquad (III)$$

où $R_1$ et $R_8$ sont tels que définis ci-dessus, ou

ii) lorsque $R_3$ dans le produit doit représenter un radical $—CH(OR_4)_2$, réaction d'un composé correspondant de formule I où $R_3$ représente un radical —CHO avec un alcool $R_4OH$,

b) la préparation d'un composé de formule I où $R_3$ représente un radical —CHO par hydrolyse sélective d'un composé correspondant de formule I où $R_3$ représente un radical $—CH(OR_4)_2$,

c) la préparation d'un composé de formule I où $R_3$ représente un radical —CH=NOH par réaction d'un composé correspondant de formule I où $R_3$ représente un radical —CHO avec l'hydroxylamine ou un sel de celle-ci,

d) la préparation d'un composé de formule I où $R_3$ représente un radical —CN par déshydratation d'un composé correspondant de formule I où $R_3$ représente un radical —CH=NOH,

e) la préparation d'un composé de formule I où $R_3$ représente un radical $—CH_2OH$ par réduction sélective d'un composé correspondant de formule I où $R_3$ représente un radical —CHO,

f) la préparation d'un composé de formule I où $R_2$ représente un atome d'hydrogène par hydrolyse sélective d'un composé correspondant de formule I où $—COOR_2$ représente un radical ester,

g) la préparation d'un composé de formule I où $R_2$ représente autre chose qu'un atome d'hydrogène par estérification, à l'aide d'un composé $R_2L$ où L représente un radical partant, d'un composé correspondant de formule I où $R_2$ représente un atome d'hydrogène ou bien $R_2$ conjointement $R_3$ forme une chaîne avec —CHOH—, ou

h) la réduction sélective d'une pyridine correspondante,

et, lorsque la chose est souhaitée ou nécessaire, la conversion du composé résultant de formule I en un sel d'addition d'acide pharmaceutiquement acceptable de celui-ci, et réciproquement.

2. Procédé suivant la revendication 1, dans lequel X représente un atome d'oxygène et le radical benzofurazanyle est uni par sa position 4 au cycle tétrahydropyridine.

3. Procédé suivant la revendication 2, dans lequel $R_1$ et $R_2$ sont choisis parmi les radicaux éthyle et isopropyle.

4. Procédé suivant l'une quelconque des revendications précédentes, dans lequel $R_3$ représente un radical $—CH_2OH$, —CHO ou —CN.

5. Procédé suivant la revendication 4, dans lequel $R_3$ représente un radical —CHO ou —CN.

6. Procédé suivant l'une quelconque des revendications précédentes, dans lequel $R_8$ représente un radical méthyle.

7. Procédé suivant la revendication 1, dans lequel le radical benzofurazanyle ou benzothiadiazolyle est uni à l'entité dihydropyridine par sa position 4,

$R_1$ et $R_2$, qui peuvent être identiques ou différents, représentent chacun un radical alcoyle en C 1 à 6, et

$R_3$ représente un radical $—CH_2OH$, —CN, $—CH(OR_4)_2$, —CHO ou —CH=NOH, et

$R_8$ représente un radical méthyle.

8. Procédé suivant la revendication 1, dans lequel le radical benzofurazanyle ou benzothiodiazolyle est uni à l'entité dihydropyridine par sa position 4,

$R_3$ représente un radical $—CH_2OH$, —CN, $—(CH(OR_4)_2$, —CHO, —CH=NOH, ou, conjointement avec $R_2$, forme une chaîne —CHOH—, et

$R_8$ représente un radical méthyle,

et des sels pharmaceutiquement acceptables de ceux de ces composés qui contiennent un radical $—(CH_2)_nNR_6R_7$.

9. Procédé suivant la revendication 7, dans lequel X représente un atome d'oxygène, et

$R_3$ représente un radical —CN, —CH=NOH, —CHO ou $—CH(OR_4)_2$.

10. Procédé suivant la revendication 7, dans lequel X représente un atome d'oxygène, et $R_3$ représente le radical —CN.

11. Procédé suivant la revendication 7, dans lequel X représente un atome d'oxygène, $R_3$ représente un radical —CN, l'un d'entre $R_1$ et $R_2$ représente un radical méthyle, et l'autre représente un radical isopropyle.

12. Procédé suivant la revendication 1, dans lequel le composé de formule I est

le 4-(4-benzofurazanyl)-2-(diéthoxyméthyl)-1,4-dihydro-6-méthylpyridine-3,5-dicarboxylate de diéthyle,

le 4-(4-benzofurazanyl)-1,4-dihydro-2-(diméthoxyméthyl)-6-méthylpyridine-3,5-dicarboxylate de diméthyle,

le 4-(4-benzofurazanyl)-2-(diéthoxyméthyl)-1,4-dihydro-6-méthylpyridine-3,5-dicarboxylate de 3-éthyle et de 5-(1-méthyléthyle),

le 4-[4-(2,1,3-benzothiadiazolyl)]-2-(diéthoxyméthyl)-1,4-dihydro-6-méthylpyridine-3,5-dicarboxylate de diéthyle,

le 4-(4-benzofurazanyl)-1,4-dihydro-2-formyl-6-méthylpyridine-3,5-dicarboxylate de diéthyle,

le 4-(4-benzofurazanyl)-1,4-dihydro-2-formyl-6-méthylpyridine-3,5-dicarboxylate de diméthyle,

le 4-(4-benzofurazanyl)-1,4-dihydro-2-formyl-6-méthylpyridine-3,5-dicarboxylate de 3-éthyle et de 5-(1-méthyléthyle),

le 4-[4-(2,1,3-benzothiadiazolyl)]-1,4-dihydro-2-formyl-6-méthylpyridine-3,5-dicarboxylate de diéthyle,

le 4-(4-benzofurazanyl)-1,4-dihydro-2-formyl-6-méthylpyridine-3,5-dicarboxylate de 3-éthyle et de 5-(2-méthylpropyle),

le chlorhydrate de 4-(4-benzofurazanyl)-1,4-dihydro-2-formyl-6-méthylpyridine-3,5-dicarboxylate de 3-éthyle et de 5-(2-(N-méthyl-N-(phénylméthyl)amino)éthyle,

le 4-(4-benzofurazanyl)-1,4-dihydro-6-méthyl-2-[bis(1-méthyléthoxy)-méthyl]pyridine-3,5-dicarboxylate de 3-éthyle et de 5-(1-méthyléthyle),

l'ester 5-méthylique d'acide 4-(4-benzofurazanyl)-1,4-dihydro-2-formyl-6-méthylpyridine-3,5-dicarboxylique,

le 4-(4-benzofurazanyl)-1,4,5,7-tétrahydro-7-hydroxy-5-oxo-2-méthylfuro[3,4-b]pyridine-3-carboxylate de méthyle,

le 4-(4-benzofurazanyl)-1,4-dihydro-2-formyl-6-méthylpyridine-3,5-dicarboxylate de 5-méthyle et de 3-(1-méthyléthyle),

le 4-(4-benzofurazanyl)-1,4-dihydro-2-formyl-6-méthylpyridine-3,5-dicarboxylate de 5-méthyle et de 3-(2-phénoxyéthyle),

le 4-(4-benzofurazanyl)-1,4-dihydro-2-formyl-6-méthylpyridine-3,5-dicarboxylate de 3-(2-éthoxyéthyle) et de 5-méthyle,

le chlorhydrate de 4-(4-benzofurazanyl)-1,4-dihydro-2-formyl-6-méthylpyridine-3,5-dicarboxylate de 3-(2-(N,N-diéthylamino)éthyle et de 5-méthyle,

le 4-(4-benzofurazanyl)-1,4-dihydro-2-(hydroxyméthyl)-6-méthylpyridine-3,5-dicarboxylate de diéthyle'

le 4-(4-benzofurazanyl)-1,4-dihydro-2-(hydroxyméthyl)-6-méthylpyridine-3,5-dicarboxylate de diméthyle,

le 4-(4-benzofurazanyl)-1,4-dihydro-2-(hydroxyméthyl)-6-méthylpyridine-3,5-dicarboxylate de 3-éthyle et de 5-(1-méthyléthyle),

le 4-[4-(2,1,3-benzothiadiazolyl)]-1,4-dihydro-2-(hydroxyméthyl)-6-méthylpyridine-3,5-dicarboxylate de diéthyle,

le 4-(4-benzofurazanyl)-1,4-dihydro-2-(hydroxyméthyl)-6-méthylpyridine-3,5-dicarboxylate de 3-éthyle et de 5-(2-méthylpropyle),

le 4-(4-benzofurazanyl)-2-cyano-1,4-dihydro-6-méthylpyridine-3,5-dicarboxylate de diéthyle,

le 4-(4-benzofurazanyl)-2-cyano-1,4-dihydro-6-méthylpyridine-3,5-dicarboxylate de diméthyle,

le 4-(4-benzofurazanyl)-2-cyano-1,4-dihydro-6-méthylpyridine-3,5-dicarboxylate de 3-éthyle et de 5-(1-méthyléthyle),

le 4-[4-(2,1,3-benzothiadiazolyl)]-2-cyano-1,4-dihydro-6-méthylpyridine-3,5-dicarboxylate de diéthyle,

le 4-(4-benzofurazanyl)-2-cyano-1,4-dihydro-6-méthylpyridine-3,5-dicarboxylate de 3-éthyle et de 5-(2-méthylpropyle),

le 4-(4-benzofurazanyl)-1,4-dihydro-2-(hydroxyiminométhyl)-6-méthylpyridine-3,5-dicarboxylate de 3-éthyle et de 5-(2-méthylpropyle), ou

le 4-(4-benzofurazanyl)-1,4-dihydro-2-méthyl-6-(trifluorométhyl)-pyridine-3,5-dicarboxylate de diéthyle.